# EUROPEAN PATENT SPECIFICATION

(11) **EP 1 146 886 B1**
(45) Date of publication and mention of the grant of the patent: **24.07.2002**
(21) Application number: 00905004.8
(22) Date of filing: 02.02.2000
(51) Int. Cl.: A61K 35/78, A61P 17/00

(54) **SOYA EXTRACT CONTAINING LIPIDS, PROCESS FOR ITS PRODUCTION, AND PHARMACEUTICAL AND COSMETIC COMPOSITION**
LIPIDE ENTHALTENDE SOJAEXTRAKTE, VERFAHREN ZU DEREN HERSTELLUNG UND PHARMAZEUTISCHE UND KOSMETISCHE ZUSAMMENSETZUNG
EXTRAIT DE SOJA RENFERMANT DES LIPIDES ET SON PROCEDE DE FABRICATION; COMPOSITION PHARMACEUTIQUE ET COSMETIQUE

(30) Priority: 03.02.1999 DE 19904352
(43) Date of publication of application: 24.10.2001
(73) Proprietor: INDENA S.p.A., 20139 Milano (IT)
(72) Inventor: BOMBARDELLI, Ezio, I-20141 Milano (IT)
(74) Representative: Zumstein, Fritz, Dr.
(86) International application number: EP0000826
(87) International publication number: WO0045828

(56) References cited:
- WO-A-92/17160
- FR-A- 2 692 781
- DATABASE WPI Section Ch, Week 199437 Derwent Publications Ltd., London, GB; Class B05, AN 1994-299792 XP002139444 & JP 06 228171 A (TAIYO KAGAKU KK), 16 August 1994 (1994-08-16) cited in the application

## Description

In JP 06228171-A, a purification of sphingomyelin by contacting soybean with non-polar carrier and extracting sphingomyelin from non-polar carrier with a polar solvent is described.

The invention relates to novel extracts obtained by extraction of ripe whole soya beans or from oil-free soya flour (Glycine max (L.) MERRIL, Leguminosae family), to their production and to formulations containing these extracts. The novel extracts are characterized by their content of sphingomyelins and phospholipids in defined ratios. In this context, "sphingomyelins" are taken to mean phospholipids derived from sphingosine and "phospholipids" are taken to mean phospholipids derived from glycerol.

It has been found that these extracts have significant effectiveness especially in the cosmetics field for the treatment of dry skin.

Accordingly, the present invention relates to a novel soya extract which contains from about 5 to 15% by weight, preferably about 10% by weight, of sphingomyelins and from about 15 to 30% by weight, preferably above 20% by weight, of phospholipids. The sphingomyelins and the phospholipids are correspondingly present in the extract according to the invention in a ratio of from about 0.5 to 1.5 parts by weight of sphingomyelins to from about 1.5 to 3 parts by weight of phospholipids. Minor components of the extract are some sterols and long-chain fatty alcohols. In addition to the long-chain fatty alcohols and the sterols, further components of the extract are mono- and diglycerides.

The invention further relates to a process for the production of the above-defined extract, which is characterized by the following stages:
a) extraction of ripe whole soya beans or oil-free soya flour using aliphatic alcohols or a mixture of these alcohols with water;
b) concentration of the extract from stage a);
c) extraction of the concentrated extract from stage b) by treatment with aliphatic hydrocarbons;
d) treatment with vegetable activated carbon and concentration of the extract from stage c) by evaporation;
e) insolubilization of the active components using aliphatic ketones;
f) filtration of the active components, and drying.

In particular, the extracts according to the invention can be produced by extracting ripe whole soya beans or oil-free soya flour using aliphatic alcohols alone or in a mixture with water, preferably using an ethanol/water mixture, such as 95% strength ethanol. Following concentration, the extract is further extracted by treatment with aliphatic hydrocarbons. Suitable aliphatic hydrocarbons for this purpose are n-hexane and n-heptane. Following treatment with vegetable activated carbon and concentration by evaporation to a reduced volume, the organic phase is dried under reduced pressure. The active components are then rendered insoluble in aliphatic ketones. An aliphatic ketone which is suitable for this purpose is, for example, acetone. Following concentration by evaporation to a reduced volume, the organic phase is dried under reduced pressure.

Preferred conditions for carrying out the individual process stages of the process according to the invention are those given below. Here and hereinafter the unit of measurement for parts by volume is l (litre) and that for parts by weight is kg (kilogram).

Stage a: The plant starting material is selected with regard to the desired ratio of sphingomyelins to phospholipids. For this purpose, a soya flour with a predetermined lipid content is for example used as starting material. The plant material is preferably extracted with from 12 to 17 volumes of solvent per part (by weight) of biomass. The extraction temperature is expediently above 55°C. Each extraction is expediently carried out over a period of less than 4 hours. Apart from ethanol, suitable solvents are inter alia methanol, propanol and isopropanol. These solvents can contain up to 10% of water.

Stage b: The extract is expediently concentrated at a temperature below 50°C under reduced pressure. The extract is expediently concentrated by evaporation to an alcohol content of from 65 to 75%.

Stage c: The concentrated extract from stage b is expediently extracted with from 0.3 to 0.6 volumes of aliphatic hydrocarbons per part (by weight) of plant material. Extraction is preferably carried out three times.

Stage d: The organic phase is treated with vegetable activated carbon and expediently evaporated to dryness at a temperature below 50°C under reduced pressure.

Stage e: The active compounds are expediently rendered insoluble using 10 volumes of aliphatic ketones, calculated on one part (by weight) of plant material.

Stage f: The precipitate is preferably washed, for example three times; a ketone such as acetone is suitable for this purpose. Following filtration, the precipitate is expediently dried at a temperature below 50°C under reduced pressure.

The extract according to the invention is in the form of a fat-like solid, which is readily soluble in ether and chlorinated solvents.

The content of sphingomyelins in the present extract is determined using HPLC in combination with enzymatic hydrolysis of the sphingomyelins using sphingomyelinase. The latter is an enzyme which converts the sphingomyelins into ceramides, which can be detected by HPLC or TLC.

Compared with extracts known hitherto, the extracts according to the invention are distinguished by their action on dry skin.

Table 1 shows the results obtained when dry skin was treated with the extract according to the invention, compared with a collagen treatment. The treatment according to the invention was with an emulsion, in water, containing the extract according to the invention and 2% hydroxyethylcellulose.

**Table 1**

| **Treatment** | **Hydration (units)** | | **Change %** |
|---|---|---|---|
| | **Starting value** | **After 60 min** | |
| Control | 43.7±0.3 | 44.1±0.4 | - |
| 1.5% solution of the extract according to Example 1 | 44.9±0.3 | 78.9±0.3** | 75.7 |
| 2% collagen (as comparison) | 42.7±0.4 | 51.7±0.1* | 35.6 |

| | | | |
|---|---|---|---|
| *P < 0.05 | | | |
| **P < 0.001 Student t test for paired data | | | |

Skin hydration was measured as electric capacitance of the horny layer using a commercially available device (Corneometer CM 820, Courage+ Kazaka, Cologne, Germany). The mean value of five consecutive measurements was determined and recorded as the measured value. The electric capacitance is expressed by means of an arbitrary scale ranging from 0 to 100 units. Groups of 10 subjects were used.

The invention thus also extends to a pharmaceutical and cosmetic composition which contains the above extract as active component. In particular, the invention relates to a cosmetic composition which contains this extract for the prevention or treatment of dry skin.

The products or extracts according to the invention can be formulated in a suitable manner as fluid emulsion, oil/water emulsion, water/oil emulsion, lipogel, lipstick, granular powders for the production of ready-to-use solutions or fluids or liquids which are compatible with their solubility. The concentration of the extract according to the invention introduced into the formulation is in the range 1 to 5%, preferably from 1.2 to 3.5%.

The following example illustrates the invention.

### Example 1 - Production of soya extract containing 10% by weight of sphingomyelins and 20% by weight of phospholipids

10 kg of oil-free soya flour are refluxed five times with 30 litres of 95% strength ethyl alcohol. The alcohol extracts are mixed and concentrated by evaporation under reduced pressure to 5 litres. The concentrate is extracted three times with 5 litres of n-hexane. The hexane phase is treated with vegetable activated carbon and concentrated by evaporation under reduced pressure to a reduced volume. The resulting extract is then treated with 10 volumes of acetone. The sphingomyelins and phospholipids precipitate out. Following filtration, the product is dried under reduced pressure to give 220 g of extract containing 10% by weight of sphingomyelins and 20% by weight of phospholipids.

## Claims

1. Soya extract **characterized by** a content of from 5 to 15% by weight of sphingomyelins and from 15 to 30% by weight of phospholipids.

2. Soya extract according to Claim 1, **characterized by** a content of about 10% by weight of sphingomyelins and about 20% by weight of phospholipids.

3. Process for the production of an extract according to Claim 1 or 2, **characterized by** the following stages:
a) extraction of ripe whole soya beans or oil-free soya flour using aliphatic alcohols or a mixture of these alcohols with water;
b) concentration of the extract from stage a);
c) extraction of the concentrated extract from stage b) by treatment with aliphatic hydrocarbons;
d) treatment with vegetable activated carbon and concentration of the extract from stage c) by evaporation;
e) insolubilization of the active components using aliphatic ketones;
f) filtration of the active components, and drying.

4. Pharmaceutical and cosmetic composition containing, as active ingredient, an extract according to Claim 1 or 2.

5. Composition according to Claim 4 for the prevention or treatment of dry skin.

## Patentansprüche

1. Sojaextrakt, **gekennzeichnet durch** einen Gehalt an 5 bis 15 Gew.-% Sphingomyelinen und an 15 bis 30 Gew.-% Phospholipiden.

2. Sojaextrakt gemäß Anspruch 1, **gekennzeichnet durch** einen Gehalt an etwa 10 Gew.-% Sphingomyelinen und etwa 20 Gew.-% Phospholipiden.

3. Verfahren zur Herstellung eines Extrakts gemäß Anspruch 1 oder 2, **gekennzeichnet durch** die folgenden Stufen:
a) Extraktion von reifen, vollständigen Sojabohnen oder ölfreiem Sojamehl unter Verwendung von aliphatischen Alkoholen oder einer Mischung dieser Alkohole mit Wasser;
b) Konzentrieren des Extrakts von Stufe a);
c) Extrahieren des konzentrierten Extrakts von Stufe b) **durch** Behandlung mit aliphatischen Kohlenwasserstoffen;
d) Behandlung mit pflanzlicher Aktivkohle und Konzentrieren des Extrakts von Stufe c) **durch** Verdampfen;
e) Unlöslichmachen der aktiven Bestandteile unter Verwendung von aliphatischen Ketonen;
f) Filtrieren der aktiven Bestandteile und Trocknen.

4. Pharmazeutische und kosmetische Zusammensetzung, enthaltend als wirksamen Bestandteil einen Extrakt gemäß Anspruch 1 oder 2.

5. Zusammensetzung gemäß Anspruch 4 zur Vorbeugung vor oder Behandlung von trockener Haut.

## Revendications

1. Extrait de soja **caractérisé par** une teneur de 5 à 15% en poids de sphingomyélines et de 15 à 30% en poids de phospholipides.

2. Extrait de soja selon la revendication 1, **caractérisé par** une teneur d'environ 10% en poids de sphingomyélines et d'environ 20% en poids de phospholipides.

3. Procédé pour la production d'un extrait selon la revendication 1 ou 2, **caractérisé par** les étapes suivantes :
a) extraction des graines de soja entières mûres ou de farine de soja dégraissée en utilisant des alcools aliphatiques ou un mélange de ces alcools avec de l'eau ;
b) concentration de l'extrait de l'étape a) ;
c) extraction de l'extrait concentré de l'étape b) par traitement avec des hydrocarbures aliphatiques ;
d) traitement avec du charbon activé végétal et concentration de l'extrait de l'étape c) par évaporation ;
e) insolubilisation des composants actifs en utilisant des cétones aliphatiques ;
f) filtration des composants actifs, et séchage.

4. Composition pharmaceutique et cosmétique contenant, comme principe actif, un extrait selon la revendication 1 ou 2.

5. Composition selon la revendication 4 pour la prévention et le traitement des peaux sèches.
